# EUROPEAN PATENT SPECIFICATION

(11) **EP 1 513 491 B1**
(45) Date of publication and mention of the grant of the patent: **18.02.2009**
(21) Application number: 03739072.1
(22) Date of filing: 10.06.2003
(51) Int. Cl.: A61Q 1/02, A61K 8/06, A61Q 19/00, A61K 8/19

(54) **MULTI-STEP COSMETIC BENEFIT FOUNDATION KIT AND ASSOCIATED METHODS**
MEHRSTUFIGE KOSMETISCHE GRUNDIERUNGS-MAKE-UP KIT UND ENTSPRECHENDES VERFAHREN
TROUSSE DE FOND DE TEINT A EFFET COSMETIQUE BENEFIQUE EN PLUSIEURS ETAPES ET PROCEDES ASSOCIES

(30) Priority: 17.06.2002 US 389412 P
(43) Date of publication of application: 16.03.2005
(73) Proprietor: THE PROCTER & GAMBLE COMPANY, Cincinnati, Ohio 45202 (US)
(72) Inventor: RABE, Thomas, Elliot, Baltimore, MD 21208 (US); WILDGUST, Paul, Graham, Baltimore, MD 21224 (US)
(74) Representative: Wilding, Richard Alan
(86) International application number: PCT/US2003/018155
(87) International publication number: WO 2003/105787

(56) References cited:
- EP-A- 0 283 165
- FR-A- 2 686 510
- FR-A- 2 811 546
- US-A- 6 060 547
- US-B1- 6 367 484

## Description

### FIELD OF INVENTION

The present invention relates to a cosmetic kit that is suitable for use as a multi-step facial foundation product. In particular, the cosmetic kit includes at least two compositions that are topically and sequentially applied to facial skin.

### BACKGROUND

It is well known in the skin beauty care field that cosmetic benefit agents may be topically applied to human skin due to incorporation of such agents in a color cosmetic product. There are as number of benefit agents that can be applied to the skin in this manner for varying purposes including, but not limited to, absorbents, anti-acne actives, antiperspirant actives, anti-wrinkle actives, artificial tanning agents, astringents, film forming agents, hydrophilic conditioning agents, hydrophobic conditioning agents, light diffusers, desquamating agents, skin lightening agents, skin soothing/healing agents, skin thickeners, sunscreen actives, vitamin compounds, yellowness-reducing particles, redness-reducing particles, and combinations thereof. There is, however, a common problem that arises when any of these actives are incorporated into a color cosmetic. In particular, the amount of the active that can be incorporated into the color cosmetic is limited by a number of parameters, including, but not limited to, solubility, skin feel, and the ability to retain a solid to fluid ratio that yields provides a pleasant cosmetic application experience.

US6060547 describes a cosmetic composition for application as a multi-step facial foundation product, comprising a cosmetic benefit agent and one or more colorant. The foundation product is made of a water-in-oil emulsion and more specifically a water-in-silicone emulsion.

FR2686510 refers to water-in-oil stable emulsion suitable for cosmetic compositions.

FR2811546 describes a cosmetic kit suitable for application as a multi-step facial foundation product characterized by a first composition comprising a cosmetic benefit agent in a cosmetically acceptable carrier and a second composition comprising one or more colorants in an acceptable cosmetic acceptable carrier.

US6367484 relates to a cosmetic make-up kit comprising film-forming compositions.

Applicants have surprisingly found that it is possible to increase the amount of cosmetic benefit agents that are effectively delivered to the skin by utilizing a cosmetic kit that includes a first composition that includes a safe and effective amount of a cosmetic benefit agent and suitable carrier and a second composition that includes one or more high refractive index colorants and a carrier for the colorant. Applicants have also found that the first application can improve the application properties of the pigmented second composition.

Without being limited by theory, Applicants believe that this multi-compositional approach allows for improved efficiency of delivery of active ingredients through separating the skin active benefit functionality from the primary function of imparting color to the skin.

### SUMMARY OF THE INVENTION

The present invention relates to a cosmetic kit suitable for application as a multi-step facial foundation product wherein the kit comprises:
1. a first composition that comprises:
   a. a safe and effective amount of a first cosmetic benefit agent; and
   b. a first cosmetically acceptable carrier; and
2. a second composition that comprises:
   a. a safe and effective amount of one or more colorants having a refractive index greater than about 2.0; and
   b. a second cosmetically acceptable carrier;
wherein said second composition is topically applied to facial skin after said first composition,
wherein said second composition is topically applied to facial skin after said first composition, wherein the first composition is an oil-in-water emulsion, characterized in that the second competition is a water-in-silicone emulsion.

### DETAILED DESCRIPTION OF THE INVENTION

The present invention relates to a cosmetic kit suitable for application as a multi-step facial foundation product. The essential components of the kit are described below. Also included is a nonexclusive description of various optional and preferred components useful in embodiments of the present invention.

As used herein, "safe and effective amount" means an amount of a compound, component, or composition (as applicable) sufficient to significantly induce a positive effect (e.g., confer a noticeable cosmetic benefit), but low enough to avoid serious side effects, (e.g., undue toxicity or allergic reaction), i.e., to provide a reasonable benefit to risk ratio, within the scope of sound medical judgment.

As used herein, "cosmetic benefit agent" means a compound, material, and/or active that confers an aesthetic feature to the surface, preferably skin, to which it is applied.

The present invention can comprise, consist of, or consist essentially of any of the required or optional ingredients and/or limitations described herein.

All percentages and ratios are calculated on a weight basis unless otherwise indicated. All percentages are calculated based upon the total composition unless otherwise indicated.

All molar weights are weight average molecular weights and are given in units of grams per mole.

All ingredient levels are exclusive of solvents, by-products, or other impurities that may be present in commercially available sources, unless otherwise indicated. All measurements made are at ambient room temperature, which is approximately 73°F, unless otherwise designated.

### COSMETIC KIT

The cosmetic kit of the present invention includes a first composition that comprises a safe and effective amount of a first cosmetic benefit agent and a first cosmetically-acceptable carrier and a second composition that comprises a safe and effective amount of one or more colorants having a refractive index greater than about 2.0 and a second cosmetically-acceptable carrier wherein the second composition in topically applied to facial skin after the first composition.

### FIRST COMPOSITION

The first composition of the presently claimed kit is topically applied to the facial skin of a user in order to improve the condition of the skin. One benefit that the application of the first composition provides is to prepare the skin's surface for application of a smooth, natural-appearing, and aesthetically-pleasing color layer such that a consumer-acceptable facial color product is achieved. Yet another benefit that the application of the first composition may provide is delivery to the facial skin of one or more of a first cosmetic benefit agent. The first composition thereby prepares the surface of the skin for the application of the second, high refractive index color-containing composition. It is believed that this surface preparation occurs via deposition and adhesion of the first cosmetic benefit agent to the skin. It is intended, however, that the same or similar cosmetic benefit agents could be included in the second composition or any other additional compositions that are sequentially applied to the skin and packaged with the kit. Suitable cosmetic agents include, but are not limited to those selected from the group consisting of absorbents, anti-acne actives, antiperspirant actives, anti-wrinkle actives, artificial tanning agents, astringents, film forming agents, hydrophilic conditioning agents, hydrophobic conditioning agents, light diffusers, desquamating agents, skin lightening agents, skin soothing/healing agents, skin thickeners,-sunscreen actives, vitamin compounds, and combinations thereof.

In preferred embodiments of the present invention, the first and/or second compositions comprise from 0.1% to 60.0%, by weight of the composition, of a cosmetic benefit agent, whether it is in the first or second composition. In fact, even more preferred amounts of the benefit agent in increasing order of preference are from 0.5% to 50.0%, 0.5% to 40.0%, 0.5% to 35.0%, and 0.5% to 30.0%, by weight of the composition. Suitable cosmetic benefit agents include but are not limited to those that follow.

### Absorbents

The compositions of the present invention may comprise as a cosmetic benefit agent one or more absorbents. These absorbents are useful for achieving the uptake of various fluids that are commonly present on the skin, e.g., perspiration, oil, and/or sebum. Suitable absorbents include, but are not limited to, silicas, silicates, polyacrylates, cross-linked silicones, cross-linked hydrocarbons, activated carbon, starch-based materials (for example cornstarch (topical starch), talc, rice starch, oat starch, tapioca starch, potato starch, legume starches, soy starch, turnip starch), microcrystalline cellulose (for example Avicel®), aluminum starch octenyl succinate (sold by National Starch & Chemical Co. as Dry Flo® Pure, Dry Flo® XT, Dry Flo® PC, and/or Dry Flo® AF (aluminum free grade)), kaolin, calcium silicate, amorphous silicas, calcium carbonate, magnesium carbonate, or zinc carbonate, and mixtures thereof. Somespecific examples of the silicates and carbonates useful in the present invention are more fullyexplained in Van Nostrand Reinhold's Encyclopedia of Chemist[y. 4th Ed. pages 155, 169,556, and 849, (1984).

### Anti-Acne Actives

Examples of useful anti-acne actives as the cosmetic benefit agents of the present invention include, but are not limited to, the keratolytics such as salicylic acid (o-hydroxybenzoic acid), derivatives of salicylic acid such as 5-octanoyl salicylic acid, and resorcinol; retinoids such as retinoic acid and its derivatives (e.g., cis and trans); sulfur-containing D and L amino acids and their derivatives and salts, particularly their N-acetyl derivatives, a preferred example of which is N-acetyl-L-cysteine; lipoic acid; antibiotics and antimicrobials such as benzoyl peroxide, octopirox, tetracycline, 2,4,4-trichloro-2'-hydroxy diphenyl ether, 3,4,4'-trichlorobanilide, azelaic acid and its derivatives, phenoxyethanol, phenoxypropanol, phenoxyisopropanol, ethyl acetate, clindamycin and meclocycline; sebostats such as flavonoids; and bile salts such as scymnol sulfate and its derivatives, deoxycholate, and cholate.

### Antiperspirant Actives

Antiperspirant actives may also be included as the cosmetic benefit agent in the compositions of the present invention. Suitable antiperspirant actives include astringent metallic salts, especially the inorganic and organic salts of aluminum zirconium and zinc, was well as mixtures thereof. Particularly preferred are the aluminum containing and/or zirconium-containing materials or salts, such as aluminum halides, aluminum chlorohydrate, aluminum hydroxyhalides, zirconyl oxyhalides, zirconyl hydroxyhalides, and mixtures thereof.

### Anti-Wrinkle and Anti-Skin Atrophy Actives

Examples of anti-wrinkle and anti-skin atrophy actives useful as the cosmetic benefit agents of the present invention include, but are not limited to, retinoic acid and its derivatives (e.g., cis and trans); retinol; retinyl esters; niacinamide, and derivatives thereof; sulfur-containing D and L amino acids and their derivatives and salts, particularly the N-acetyl derivatives, a preferred example of which is N-acetyl-L-cysteine; thiols, e.g., ethane thiol; terpene alcohols (e.g., famesol); hydroxy acids, phytic acid, lipoic acid; lysophosphatidic acid, alpha-hydroxy acids (e.g., lactic acid and glycolic acid), beta-hydroxy acids (e.g., salicylic acid), and skin peel agents (e.g., phenol and the like).

### Artificial Tanning Actives and Accelerators

Examples of artificial tanning actives and accelerators useful in the compositions of the present invention include, but are not limited to, dihydroxyacetaone, tyrosine, tyrosine esters such as ethyl tyrosinate, and phospho-DOPA.

### Astringents

The compositions of the present invention may include astringents. Astringents are useful for shrinking pores of the skin. Suitable astringents include, but are not limited to, clove oil, fomes officinalis extract, spiraea ulmaria extract, menthol, camphor, eucalyptus oil, eugenol, menthyl lactate, witch hazel distillate, aluminum salts, tannins, ethanol, and combinations thereof.

### Film Forming Agents

Examples of suitable film forming agents useful in the compositions of the present kit include:
a) sulfopolyester resins, such as AQ sulfopolyester resins, such as AQ29D, AQ35S, AQ38D, AQ38S, AQ48S, and AQ55S (available from Eastman Chemicals);
b) polyvinylacetate/polyvinyl alcohol polymers, such as Vinex resins available from Air Products, including Vinex 2034, Vinex 2144, and Vinex 2019;
c) acrylic resins, including water dispersible acrylic resins available from National Starch under the trade name "Dermacryl", including Dermacryl LT;
d) polyvinylpyrrolidones (PVP), including Luviskol K17, K30 and K90 (available from BASF), water soluble copolymers of PVP, including PVP/VA S-630 and W-735 and PVP/dimethylaminoethylinethacrylate Copolymers such as Copolymer 845 and Copolymer 937 available from ISP, as well as other PVP polymers disclosed by E.S. Barabas in the Encyclopedia of Polymer-Science and Engineering, 2 Ed., Vol. 17, pp. 198-257;
e) high molecular weight silicones such as dimethicone and organic-substituted dimethicones, especially those with viscosities of greater than about 50,000 mPas;
f) high molecular weight hydrocarbon polymers with viscosities of greater than about 50,000 mPas;
g) silicone-acrylate copolymers, including VS-70 (3M), SA-70 (3M), KP-545 (Shin-Etsu)
h) organosiloxanes, including organosiloxane resins, fluid diorganopolysiloxane polymers and silicone ester waxes;
i) polyurethanes, including Polyderm series of polymers from Alzo, Corp.; and
j) hydrophobic acrylate copolymers, including the acrylate/alkylmethacrylate copolymer Lipacryl (Rohm & Haas) or it's emulsified, water dispersible version Allianz OPT (ISP).

Examples of these polymers and cosmetic compositions containing them are found in PCT publication Nos. WO96/33689, published 10/31/96; WO97/17058, published 5/15/97; and US Patent No. 5,505,937 issued to Castrogiovanni et al. 4/9/96. Additional film forming polymers suitable for use herein include the water-insoluble polymer materials in aqueous emulsion and water soluble film forming polymers described in PCT publication No. WO98/18431, which published 5/7/98. Examples of high molecular weight hydrocarbon polymers with viscosities of greater than about 50,000 mPas include polybutene, polybutene terephthalate, polydecene, polycyclopentadiene, and similar linear and branched high molecular weight hydrocarbons.

Preferred film forming polymers include organosiloxane resins comprising combinations of R₃SiO_{1/2} "M" units, R₂SiO "D" units, RSiO_{3/2} "T" units, SiO₂ "Q" units in ratios to each other that satisfy the relationship RₙSiO(_{4-n)/2} where n is a value between 1.0 and 1.50 and R is a methyl group. Note that a small amount, up to 5%, of silanol or alkoxy functionality may also be present in the resin structure as a result of processing. The organosiloxane resins must be solid at about 25°C and have a molecular weight range of from about 1,000 to about 10,000 grams/mole. The resin is soluble in organic solvents such as toluene, xylene, isoparaffins, and cyclosiloxanes or the volatile carrier, indicating that the resin is not sufficiently crosslinked such that the resin is insoluble in the volatile carrier. Particularly preferred are resins comprising repeating monofunctional or R₃SiO_{1/2} "M" units and the quadrafunctional or SiO₂ "Q" units, otherwise known as "MQ" resins as disclosed in U.S. Patent 5,330,747, Krzysik, issued July 19, 1994. In the present invention the ratio of the "M" to "Q" functional units is preferably about 0.7 and the value of n is 1.2. Organosiloxane resins such as these are commercially available such as Wacker 803 and 804 available from Wacker Silicones Corporation of Adrian Michigan, and G. E. 1170-002 from the General Electric Company.

Other materials for enhancing wear or transfer resistance include trimethylated silica. Suitable silicas of this type and cosmetic compositions containing them are described in US Patent No. 5,800,816 issued to Brieva et al.

### Hydrophilic Conditioning Agents

The cosmetic benefit agents of the present invention can also be one or more hydrophilic conditioning agents. Nonlimiting examples of hydrophilic conditioning agents include those selected from the group consisting of polyhydric alcohols, polypropylene glycols, polyethylene glycols, ureas, pyrolidone carboxylic acids, ethoxylated and/or propoxylated C3-C6 diols and triols, alpha-hydroxy C2-C6 carboxylic acids, ethoxylated and/or propoxylated sugars, polyacrylic acid copolymers, sugars having up to about 12 carbons atoms, sugar alcohols having up to about 12 carbon atoms, and mixtures thereof. Specific examples of useful hydrophilic conditioning agents include materials such as urea; guanidine; glycolic acid and glycolate salts (e.g., ammonium and quaternary alkyl ammonium); lactic acid and lactate salts (e.g., ammonium and quaternary alkyl ammonium); sucrose, fructose, glucose, eruthrose, erythritol, sorbitol, mannitol, glycerol, hexanetriol, propylene glycol, butylene glycol, hexylene glycol, and the like; polyethylene glycols such as PEG-2, PEG-3, PEG-30, PEG-50, polypropylene glycols such as PPG-9, PPG-12, PPG-15, PPG-17, PPG-20, PPG-26, PPG-30, PPG-34; alkoxylated glucose; hyaluronic acid; cationic skin conditioning polymers (e.g., quaternary ammonium polymers such as Polyquaternium polymers); and mixtures thereof. Glycerol, in particular, is a preferred hydrophilic conditioning agent in the articles of the present invention. Also useful are materials such as aloe vera in any of its variety of forms (e.g., aloe vera gel), chitosan and chitosan derivatives, e.g., chitosan lactate, lactamide monoethanolamine; acetamide monoethanolamine; and mixtures thereof. Also useful are propoxylated glycerols as described in propoxylated glycerols described in U. S. Patent No. 4,976,953, to Orr et al., issued December 11, 1990.

### Hydrophobic Conditioning Agents

The cosmetic benefit agent may be one or more hydrophobic conditioning agents. Nonlimiting examples of hydrophobic conditioning agents include those selected from the group consisting of mineral oil, petrolatum, lecithin, hydrogenated lecithin, lanolin, lanolin derivatives, C7-C40 branched chain hydrocarbons, C1-C30 alcohol esters of C1-C30 carboxylic acids, C1-C30 alcohol esters of C2-C30 dicarboxylic acids, monoglycerides of C1-C30 carboxylic acids, diglycerides of C1-C30 carboxylic acids, triglycerides of C1-C30 carboxylic acids, ethylene glycol monoesters of C1-C30 carboxylic acids, ethylene glycol diesters of C1-C30 carboxylic acids, propylene glycol monoesters of C1-C30 carboxylic acids, propylene glycol diesters of C1-C30 carboxylic acids, C1-C30 carboxylic acid monoesters and polyesters of sugars, polydialkylsiloxanes, polydiarylsiloxanes, polyalkarylsiloxanes, cylcomethicones having 3 to 9 silicon atoms, vegetable oils, hydrogenated vegetable oils, polypropylene glycol C4-C20 alkyl ethers, di C8-C30 alkyl ethers, and combinations thereof.

Straight and branched chain hydrocarbons having from about 7 to about 40 carbon atoms are useful herein. Nonlimiting examples of these hydrocarbon materials include dodecane, isododecane, squalane, cholesterol, hydrogenated polyisobutylene, docosane (i.e. a C₂₂ hydrocarbon), hexadecane, isohexadecane (a commercially available hydrocarbon sold as Permethyl^{®} 101A by Presperse, South Plainfield, NJ). C7-C40 isoparaffins, a class of C7-C40 branched hydrocarbons, are useful herein. Polydecene, a branched liquid hydrocarbon, is also useful herein and is commercially available under the tradenames Puresyn 100® and Puresyn 3000® from Mobile Chemical (Edison, NJ).

Also useful are C1-C30 alcohol esters of C1-C30 carboxylic acids and of C2-C30 dicarboxylic acids, including straight and branched chain materials as well as aromatic derivatives. Also useful are esters such as monoglycerides of C1-C30 carboxylic acids, diglycerides of C1-C30 carboxylic acids, triglycerides of C1-C30 carboxylic acids, ethylene glycol monoesters of C1-C30 carboxylic acids, ethylene glycol diesters of C1-C30 carboxylic acids, propylene glycol monoesters of C1-C30 carboxylic acids, and propylene glycol diesters of C1-C30 carboxylic acids. Straight chain, branched chain and aryl carboxylic acids are included herein. Also useful are propoxylated and ethoxylated derivatives of these materials. Nonlimiting examples include diisopropyl sebacate, diisopropyl adipate, isopropyl myristate, isopropyl palmitate, myristyl propionate, ethylene glycol distearate, 2-ethylhexyl palmitate, isodecyl neopentanoate, di-2-ethylhexyl maleate, cetyl palpitate, myristyl myristate, stearyl stearate, cetyl stearate, behenyl behenrate, dioctyl maleate, dioctyl sebacate, diisopropyl adipate, cetyl octanoate, diisopropyl dilinoleate, carpylic/capric triglyceride, PEG-6 caprylic/capric triglyceride, PEG-8 caprylic/capric triglyceride, and combinations thereof.

Also useful are various C1-C30 monoesters and polyesters of sugars and related materials. These esters are derived from a sugar or polyol moiety and one or more carboxylic acid moieties. Depending on the constituent acid and sugar, these esters can be in either liquid or solid form at room temperature. Examples of liquid esters include: glucose tetraoleate, the glucose tetraesters of soybean oil fatty acids (unsaturated), the mannose tetraesters of mixed soybean oil fatty acids, the galactose tetraesters of oleic acid, the arabinose tetraesters of linoleic acid, xylose tetralinoleate, galactose pentaoleate, sorbitol tetraoleate, the sorbitol hexaesters of unsaturated soybean oil fatty acids, xylitol pentaoleate, sucrose tetraoleate, sucrose pentaoletate, sucrose hexaoleate, sucrose hepatoleate, sucrose octaoleate, and mixtures thereof. Examples of solid esters include: sorbitol hexaester in which the carboxylic acid ester moieties are palmitoleate and arachidate in a 1:2 molar ratio; the octaester of raffinose in which the carboxylic acid ester moieties are linoleate and behenate in a 1:3 molar ratio; the heptaester of maltose wherein the esterifying carboxylic acid moieties are sunflower seed oil fatty acids and lignocerate in a 3:4 molar ratio; the octaester of sucrose wherein the esterifying carboxylic acid moieties are oleate and behenate in a 2:6 molar ratio; and the octaester of sucrose wherein the esterifying carboxylic acid moieties are laurate, linoleate and behenate in a 1:3:4 molar ratio. A preferred solid material is sucrose polyester in which the degree of esterification is 7-8, and in which the fatty acid moieties are C18 mono- and/or di-unsaturated and behenic, in a molar ratio of unsaturates: behenic of 1:7 to 3:5. A particularly preferred solid sugar polyester is the octaester of sucrose in which there are about 7 behenic fatty acid moieties and about 1 oleic acid moiety in the molecule. Other materials include cottonseed oil or soybean oil fatty acid esters of sucrose. The ester materials are further described in, U. S, Patent No. 2,831,854, U. S. Patent No. 4,005,196, to Jandacek, issued January 25, 1977; U. S. Patent No. 4,005,195, to Jandacek, issued January 25, 1977, U. S. Patent No. 5,306,516, to Letton et al., issued April 26, 1994; U. S. Patent No. 5,306,515, to Letton et al., issued April 26, 1994; U. S. Patent No. 5,305,514, to Letton et al., issued April 26, 1994; U. S. Patent No. 4,797,300, to Jandacek et al., issued January 10, 1989; U. S. Patent No. 3,963,699, to Rizzi et al, issued June 15, 1976; U. S. Patent No. 4,518,772, to Volpenhein, issued May 21, 1985; and U. S. Patent No. 4,517,360, to Volpenhein, issued May 21, 1985.

Nonvolatile silicones such as polydialkylsiloxanes, polydiarylsiloxanes, and polyalkarylsiloxanes are also useful oils. These silicones are disclosed in U. S. Patent No. 5,069,897, to Orr, issued December 3, 1991. The polyalkylsiloxanes correspond to the general chemical formula R₃SiO[R₂SiO]ₓSiR₃ wherein R is an alkyl group (preferably R is methyl or ethyl, more preferably methyl) and x is an integer up to about 500, chosen to achieve the desired molecular weight. Commercially available polyalkylsiloxanes include the polydimethylsiloxanes, which are also known as dimethicones, nonlimiting examples of which include the Vicasil^{®} series sold by General Electric Company and the Dow Coming^{®} 200 series sold by Dow Corning Corporation. Specific examples of polydimethylsiloxanes useful herein include Dow Corning^{®} 225 fluid having a viscosity of 10 centistokes and a boiling point greater than 200°C, and Dow Corning^{®} 200 fluids having viscosities of 50, 350, and 12,500 centistokes, respectively, and boiling points greater than 200°C. Also useful are materials such as trimethylsiloxysilicate, which is a polymeric material corresponding to the general chemical formula [(CH₂)₃SiO_{1/2}]ₓ[SiO₂]_{y}, wherein x is an integer from about 1 to about 500 and y is an integer from about 1 to about 500. A commercially available trimethylsiloxysilicate is sold as a mixture with dimethicone as Dow Corning^{®} 593 fluid. Also useful herein are dimethiconols, which are hydroxy terminated dimethyl silicones. These materials can be represented by the general chemical formulas R₃SiO[R₂SiO]ₓSiR₂OH and HOR₂SiO[R₂SiO]ₓSiR₂OH wherein R is an alkyl group (preferably R is methyl or ethyl, more preferably methyl) and x is an integer up to about 500, chosen to achieve the desired molecular weight. Commercially available dimethiconols are typically sold as mixtures with dimethicone or cyclomethicone (e.g. Dow Corning^{®} 1401, 1402, and 1403, fluids). Also useful herein are polyalkylaryl siloxanes, with polymethylphenyl siloxanes having viscosities from about 15 to about 65 centistokes at 25°C being preferred. These materials are available, for example, as SF 1075 methylphenyl fluid (sold by General Electric Company) and 556 Cosmetic Grade phenyl trimethicone fluid (sold by Dow Coming Corporation). Alkylated silicones such as methyldecyl silicone and methyloctyl silicone are useful herein and are commercially available from General Electric Company. Alkyl modified siloxanes are userful as well. They include alkyl methicones and alkyl dimethicones wherein the alkyl chain contains 10 to 50 carbons. Such siloxanes are commercially available under the tradenames ABIL WAX 9810 (C₂₄-C₂₈ alkyl methicone) (sold by Goldschmidt) and SF1632 (cetearyl methicone)(sold by General Electric Company). Cyclomethicone/dimethicone copolyol mixtures are also particularly useful as formulation aid/conditioning agents. A suitable mixture is sold under the tradename DC 3225Q®.

Vegetable oils and hydrogenated vegetable oils are also useful herein. Examples of vegetable oils and hydrogenated vegetable oils include safflower oil, castor oil, coconut oil, cottonseed oil, menhaden oil, palm kernel oil, palm oil, peanut oil, soybean oil, rapeseed oil, linseed oil, rice bran oil, pine oil, sesame oil, sunflower seed oil, hydrogenated safflower oil, hydrogenated castor oil, hydrogenated coconut oil, hydrogenated cottonseed oil, hydrogenated menhaden oil, hydrogenated palm kernel oil, hydrogenated palm oil, hydrogenated peanut oil, hydrogenated soybean oil, hydrogenated rapeseed oil, hydrogenated linseed oil, hydrogenated rice bran oil, hydrogenated sesame oil, hydrogenated sunflower seed oil, and mixtures thereof.

Also useful are C4-C20 alkyl ethers of polypropylene glycols, C1-C20 carboxylic acid esters of polypropylene glycols, and di-C8-C30 alkyl ethers. Nonlimiting examples of these materials include PPG-14 butyl ether, PPG-15 stearyl ether, dioctyl ether, dodecyl octyl ether, and mixtures thereof.

Hydrophobic chelating agents are also useful herein as hydrophobic conditioning agents. Suitable agents are described in U. S. Patent No. 4,387,244, issued to Scanlon et al. on June 7, 1983, and copending U.S. Patent Application Serial Nos. 09/258,747 and 09/259,485, filed in the names of Schwartz et al. on February 26, 1999.

Preferred hydrophobic conditioning agents are selected from the group consisting of mineral oil, petrolatum, lecithin, hydrogenated lecithin, lanolin, lanolin derivatives, C7-C40 branched chain hydrocarbons, C1-C30 alcohol esters of C1-C30 carboxylic acids, C1-C30 alcohol esters of C2-C30 dicarboxylic acids, monoglycerides of C1-C30 carboxylic acids, diglycerides of C1-C30 carboxylic acids, triglycerides of C1-C30 carboxylic acids, ethylene glycol monoesters of C1-C30 carboxylic acids, ethylene glycol diesters of C1-C30 carboxylic acids, propylene glycol monoesters of C1-C30 carboxylic acids, propylene glycol diesters of C1-C30 carboxylic acids, C1-C30 carboxylic acid monoesters and polyesters of sugars, polydialkylsiloxanes, polydiarylsiloxanes, polyalkylarylsiloxanes, cylcomethicones having 3 to 9 silicon atoms, vegetable oils, hydrogenated vegetable oils, polypropylene glycol C4-C20 alkyl ethers, di C8-C30 alkyl ethers, and combinations thereof.

### Light Diffusers

The compositions of the claimed kit may include as the cosmetic benefit agent a light diffuser. Light diffusers are useful for improving skin appearance by minimizing the appearance of texture such as pores and fine lines. Suitable light diffusers for inclusion into the compositions of the present kit include, but are not limited to silica, nylon, polyethylene, polymethyl methacrylate, poystyrene, methylsiloxane copolymer, polytetrafluoroethylene copolymer, boron nitride, silicone resin powders, silicone rubber powders, ethylene acrylate copolymers, mica, titanium dioxide, zinc oxide, and combinations thereof.

### Desquamating Agents

The compositions of the present invention may include one or more desquamating agents. Such agents are useful for improving the turnover of skin in such a manner that the skin appears rejuvenated and younger looking. For example, the desquamation actives tend to improve the texture of the skin (e.g., smoothness). One desquamation system that is suitable for use herein contains sulfhydryl compounds and zwitterionic surfactants and is described in U.S. Patent No. 5,681,852, to Bissett. Another desquamation system that is suitable for use herein contains salicylic acid and zwitterionic surfactants and is described in U.S. Patent No. 5,652,228 to Bisset. Zwitterionic surfactants such as described in these applications are also useful as desquamatory agents herein, with cetyl betaine being particularly preferred.

### Skin Lightening Agents

Another suitable cosmetic benefit agent is a skin lightening agent. Suitable skin lightening agents include those known in the art, including kojic acid, arbutin, deoxyarbutin, ascorbic acid and derivatives thereof, e.g., magnesium ascorbyl phosphate or sodium ascorbyl phosphate or other salts of ascorbyl phosphate.

### Skin Soothing and Skin Healing Actives

A safe and effective amount of a skin soothing or skin healing active may be added to the present composition, preferably, from about 0.1% to about 30%, more preferably from about 0.5% to about 20%, still more preferably from about 0.5% to about 10 %, by weight of the composition formed. Skin soothing or skin healing actives suitable for use herein include panthenoic acid derivatives (including panthenol, dexpanthenol, ethyl panthenol), aloe vera, allantoin, bisabolol, and dipotassium glycyrrhizinate.

### Sunscreen Actives

Also useful herein as cosmetic benefit agents are sunscreen actives. A wide variety of sunscreen actives are described in U.S. Patent No. 5,087,445, to Haffey et al., issued February 11, 1992; U.S. Patent No. 5,073,372, to Turner et al., issued December 17, 1991; U.S. Patent No. 5,073,371, to Turner et al. issued December 17, 1991; and Sagarin, et al., at Chapter VIII, pages 189 et seq., of Cosmetics Science and Technology. Nonlimiting examples of sunscreens which are useful in the compositions of the present invention are those selected from the group consisting of 2-ethylhexyl *p*-methoxycinnamate, 2-ethylhexyl N,N-dimethyl-p-aminobenzoate, p-aminobenzoic acid, 2-phenylbenzimidazole-5-sulfonic acid, octocrylene, oxybenzone, homomenthyl salicylate, octyl salicylate, 4,4'-methoxy-t-butyldibenzoylinethane, 4-isopropyl dibenzoylmethane, 3-benzylidene camphor, 3-(4-methylbenzylidene) camphor, titanium dioxide, zinc oxide, silica, iron oxide, and mixtures thereof. Still other useful sunscreens are those disclosed in U.S. Patent No. 4,937,370, to Sabatelli, issued June 26, 1990; and U.S. Patent No. 4,999,186, to Sabatelli et al., issued March 12, 1991. Especially preferred examples of these sunscreens include those selected from the group consisting of 4-N,N-(2-ethylhexyl)methylaminobenzoic acid ester of 2,4-dihydroxybenzophenone, 4-N,N-(2-ethylhexyl)methylaminobenzoic acid ester with 4-hydroxydibenzoylmethane, 4-N,N- (2-ethylhexyl)-methylaminobenzoic acid ester of 2-hydroxy-4-(2-hydroxyethoxy)benzophenone, 4-N,N-(2-ethylhexyl)-methylaminobenzoic acid ester of 4-(2-hydroxyethoxy)dibenzoylmethane, and mixtures thereof. Exact amounts of sunscreens that can be employed will vary depending upon the sunscreen chosen and the desired Sun Protection Factor (SPF) to be achieved. SPF is a commonly used measure of photoprotection of a sunscreen against erythema.

### Vitamin Compounds

The present compositions may comprise vitamin compounds, precursors, and derivatives thereof as the cosmetic benefit agents. These vitamin compounds may be in either natural or synthetic form. Suitable vitamin compounds include, but are not limited to, Vitamin A (e.g., beta carotene, retinoic acid, retinol, retinoids, retinyl palmitate, retinyl proprionate, etc.), Vitamin B (e.g., niacin, niacinamide, riboflavin, pantothenic acid, etc.), Vitamin C (e.g., ascorbic acid, etc.), Vitamin D (e.g., ergosterol, ergocalciferol, cholecalciferol, etc.), and Vitamin E (e.g., tocopherol).

### Enzymes

The cosmetic benefit agents of the present invention may be one or more enzymes. Preferably, such enzymes are dermatologically acceptable. Suitable enzymes include, but are not limited to, keratinase, protease, amylase, subtilisin, other peptides and proteins, etc..

Peptides, including but not limited to, di-, tri-, tetra-, and pentapeptides and derivatives thereof, may be included as the cosmetic benefit agents of the present invention in amounts that are safe and effective. As used herein, "peptides" refers to both the naturally occuring peptides and synthesized peptides. Also useful herein are naturally occurring and commercially available compositions that contain peptides.

### Chelators

The cosmetic benefit agents in the compositions of the present kit may be chelators. As used herein, "chelator" or "chelating agent" means an active agent capable of removing a metal ion from a system by forming a complex so that the metal ion cannot readily participate in or catalyze chemical reactions. The inclusion of a chelating agent is especially useful for providing protection against UV radiation that can contribute to excessive scaling or skin texture changes and against other environmental agents, which can cause skin damage.

Exemplary chelators that are useful herein are disclosed in U.S. Patent No. 5,487,884, issued 1/30/96 to Bissett et al.; International Publication No. 91/16035, Bush et al., published 10/31/95; and International Publication No. 91/16034, Bush et al., published 10/31/95. Preferred chelators useful in compositions of the subject invention are furildioxime, furildioxime derivatives, furilmonoxime, furilmonoxime derivatives, and combinations thereof.

### Flavonoids

The cosmetic benefit agents of the present invention may also be a flavonoid. Flavonoids are broadly disclosed in U.S. Patents 5,686,082 and 5,686,367.

### Sterols

The cosmetic benefit agents of the present invention may also be a safe and effective amount of one or more sterol compounds. Examples of useful sterol compounds include sitosterol, stigmasterol, campesterol, brassicasterol, lanosterol, 7-dehydrocholesterol, and mixtures thereof. These can be synthetic in origin or from natural sources, e.g., blends extracted from plant sources (e.g., phytosterols).

### SECOND COMPOSITION

The second composition of the kit of the present invention comprises a safe and effective amount of one or more colorants having a refractive index greater than about 2.0 and a cosmetically-acceptable carrier. The primary purpose of this second composition is to deliver the desirable shade or color and that the user is seeking by his/her use of the facial foundation, as well as even out skin tone by covering or hiding tonal imperfections. This second composition may, however, also be instrumental in providing deposition and substantivity of additional cosmetic benefit agents to the skin's surface. For instance, in certain embodiments the second composition may contain the same or similar cosmetic benefit agents as are present in the first composition such that the amount delivered and subsequently deposited to the skin is increased due to this multi-step approach. In particular, in order to achieve a long-wearing product, film forming agents may be incorporated into both the first and second compositions as could other cosmetic benefit agents that would work synergistically together to provide an improved benefit.

The colorants of the second composition exhibit a refractive index of greater than about 2.0. Applicants have found that colorants having higher refractive indices are particularly useful in the second compositions since the primary function of the said second composition is to impart color and tonal coverage of the underlying skin. With regard to the colorants useful in the second composition of the present invention, a wide variety of colorants are suitable for use herein. For instance, suitable colorants include, but are not limited to, D&C Yellow No. 7, D&C Red No. 36, FD&C Red No. 4, D&C Orange No. 4, D&C Red No. 6, D&C Red No. 34, FD&C Yellow No. 6, D&C Red No. 33, FD&C Yellow No. 5, D&C Brown No. 1, D&C Red No. 17, FD&C Green No. 3, D&C Blue No. 4, D&C Yellow No. 8, D&C Orange No. 5, D&C Red No. 22, D&C Red No. 21, D&C Red No. 28, D&C Orange No. 11, D&C Yellow No. 10, D&C Violet No. 2, Ext. D&C Violet No. 2, D&C Green No. 6, D&C Green No. 5, D&C Red No. 30, D&C Green No. 8, D&C Red No. 7, FD&C Blue No. 1, D&C Yellow No. 7, D&C Red No. 27, D&C Orange No. 10, D&C Red No. 31, FD&C Red No. 40, D&C Yellow No. 11, Annatto extract, β carotene, guanine, carmine, aluminum powder, ultramarines, bismuth oxychloride, chromium oxide green , chromium hydroxide green, iron oxides, ferric ferrocyanide, manganese violet, titanium dioxide, titanated mica (i.e., mica coated with titanium dioxide), iron oxide titanated mica, zinc oxide, caramel coloring, mica, ferric ammonium ferrocyanide, dihydroxyacetone, guaiazulene, pyrophyllite, bronze powder, copper powder, aluminum stearate, calcium stearate, lactofavin, magnesium stearate, zinc stearate, capsanthin/capsorubin, bentonite, barium sulfate, calcium carbonate, calcium sulfate, carbon black, magnesium carbonate, magnesium silicate, colored silica, silica (including spherical silica, hydrated silica and silica beads), CI 10020, CI 11680, CI 15630, CI 15865, CI 16185, CI 16255, CI 16255, CI 45430, CI 69825, CI 73000, CI 73015, CI 74160, CI 75100, CI 77002, CI 77346, CI 77480, nylon powder, polyethylene powder, ethylene acrylates copolymer powder, methacrylate powder, polystyrene powder, silk powder, crystalline cellulose, starch, bismuth oxychloride, guanine, kaolin, chalk, diatomaceous earth, microsponges, boron nitride and the like.. Additionally, lakes or composites of these colorants may also be used. Additional colorants, pigments, and powders useful herein are described in US Patent No. 5,505,937 issued to Castrogiovanni et al. 4/9/96.

In preferred embodiments of the present invention, the second composition comprises from 0.5% to 80.0%, by weight of the composition, of a high refractive index colorant. In fact, even more preferred amounts of the colorant in the second composition, in increasing order of preference are from 0.5% to 50.0%, 0.5% to 40.0%, 1.0% to 35.0%, and 1.5% to 30.0%, by weight of the composition.

### COSMETICALLY-ACCEPTABLE CARRIERS

The first and second compositions of the present invention each comprise a cosmetically-acceptable carrier for the first cosmetic benefit agent and pigment, respectively. Suitable carriers are well known in the art and are selected based on the end use application. For example, carriers of the present invention include, but are not limited to, those suitable for application to skin. Preferably, the carriers of the present invention are suitable for application to skin (e.g., sunscreens, creams, milks, lotions, masks, serums, etc.). Such carriers are well-known to one of ordinary skill in the art, and can include one or more compatible liquid or solid filler diluents or vehicles which are suitable for application to skin. The exact amount of carrier will depend upon the level of the first cosmetic benefit agent in the first composition and the level of the pigment in the second composition as well as any optional ingredients that are included in each of the compositions that one of ordinary skill in the art would classify as distinct from the carrier (e.g., other active components). Each of the first and second compositions of the present invention preferably comprises from 5% to 99%, more preferably from 10 % to 98%, and most preferably, from 10 % to 97%, by weight of the composition, of a carrier.

The carrier and compositions herein can be formulated in a number of ways, including but not limited to emulsions. For example, suitable emulsions include oil-in-water, water-in-oil, water-in-silicone, silicone-in-water, water-in-oil-in-water, oil-in-water-in-oil, and oil-in-water-in-silicone emulsions. Preferred first compositions comprise an oil-in-water emulsion while preferred second compositions comprise a water-in-silicone emulsion.

The compositions of the present invention can be formulated into a wide variety of product types, including powders, creams, waxes, pastes, lotions, milks, mousses, gels, oils, tonics, and sprays. Preferred compositions are formulated into color cosmetic products, even more preferably facial foundations, and even more preferably, facial foundations that are in the form of liquids, lotions, creams, gels, sprays, and combinations thereof. In certain embodiments, any one of the compositions of the present kit may be electrostatically applied to the skin as described in co-pending applications Ser. Nos. 09/628,631, 09/629734, 09/629765, 09/628630, all filed on July 31, 2000 and claiming priority to May 31, 2000. Any additional components required to formulate such products vary with product type and can be routinely chosen by one skilled in the art.

### OPTIONAL INGREDIENTS

The compositions of the present invention may contain a variety of other components such as are conventionally used in a given product type provided that they do not unacceptably alter the benefits of the invention. These optional components should be suitable for application to mammalian skin, that is, when incorporated into the compositions they are suitable for use in contact with human skin without undue toxicity, incompatibility, instability, allergic response, and the like, within the scope of sound medical or formulator's judgment. The CTFA Cosmetic Ingredient Handbook, Second Edition (1992) describes a wide variety of nonlimiting cosmetic and pharmaceutical ingredients commonly used in the skin care industry, which are suitable for use in the compositions of the present invention. Suitable optional ingredients include:

### Oil-soluble Polymeric Gelling Agents

The compositions of the present invention may optionally comprise one or more polymeric materials that are oil-soluble and form a gel with hydrophobic materials (e.g., oils) that are contained in the compositions. Such polymers are beneficial for structuring these materials resulting in flexible gels with improved stability and shear-resistance.

Particularly suitable are at least partially cross-linked oil-soluble polymeric materials with a softening point < 160° C. Suitable materials come from the chemical groups of PE (polyethylenes), PVA (polyvinyl alcohols) and derivatives, PVP (polyvinylpyrrolidones) and derivatives, PVP/Alkene Copolymers, PVP/VA copolymers, PVM/MA (methyl vinyl ether/maleic anhydride) copolymers and their esters and ethers, particularly poly (alkyl vinyl ether-co-maleic anhydride) copolymers, ethylene/VA copolymers, acrylates/alkyl methacrylate, styrene/isoprene, styrene/ethylene/butylene, styrene/ethylene/propylene, styrene/ethylene/butylene/styrene, styrene/butadiene copolymers, benotnite clays, hectorite clays, organic waxes and silicone waxes. Suitable materials are available e.g. from Dupont (ELVAX® types), BASF (LUVISKOL® types), Shell (KRATON® polymers), ISP (PVP, GANTREZ®, GANEX® and ALLIANZ OPT® types) and Rohm & Haas (LIPACRYL®).).

### Hydrophilic Gelling Agent

The compositions of the invention may optionally contain a hydrophilic gelling agent at a level preferably from 0.01% to 10%, more preferably from 0.02% to 2%, and especially from 0.02% to 0.5%. The gelling agent preferably has a viscosity (1% aqueous solution, 20° C., Brookfield RVT) of at least about 4000 mPa s, more preferably at least about 10,000 mPa s and especially at least 50,000 mPa-s.

Suitable hydrophilic gelling agents can generally be described as water-soluble or colloidally water-soluble polymers, and include cellulose ethers (e.g. hydroxyethyl cellulose, methyl cellulose, hydroxypropylmethyl cellulose), bentonite clays, hectorite clays, polyvinylpyrrolidone, polyvinylalcohol, polyquatemium-10, guar gum, hydroxypropyl guar gum and xanthan gum.

Among suitable hydrophilic gelling agents are acrylic acid/ethyl acrylate copolymers and the carboxyvinyl polymers sold by the B. F. Goodrich Company under the trademark of Carbopol resins. These resins consist essentially of a colloidally water-soluble polyalkenyl polyether crosslinked polymer of acrylic acid crosslinked with from 0.75% to 2.00% of a crosslinking agent such as for example polyallyl sucrose or polyallyl pentaerythritol. Examples include Carbopol 934, Carbopol 940, Carbopol 950, Carbopol 980, Carbopol 951 and Carbopol 981. Carbopol 934 is a water-soluble polymer of acrylic acid crosslinked with about 1% of a polyallyl ether of sucrose having an average of about 5.8 allyl groups for each sucrose molecule. Also suitable for use herein are hydrophobically-modified cross-linked polymers of acrylic acid having amphipathic properties available under the Trade Name Carbopol 1382, Carbopol 1342 and Pemulen TR-1 (CTFA Designation: Acrylates/10-30 Alkyl Acrylate Crosspolymer). A combination of the polyalkenyl polyether cross-linked acrylic acid polymer and the hydrophobically modified cross-linked acrylic acid polymer is also suitable for use herein. Other suitable gelling agents suitable for use herein are oleogels such as trihydroxystearin and aluminium magnesium hydroxy stearate. The gelling agents herein are particularly valuable for providing excellent stability characteristics over both normal and elevated temperatures.

Neutralizing agents suitable for use in neutralizing acidic group containing hydrophilic gelling agents herein include sodium hydroxide, potassium hydroxide, ammonium hydroxide, monoethanolamine, diethanolamine and triethanolamine.

### Emulsifiers/Surfactants

Emulsifers (also known as "surfactants") can also be included into the compositions of the present invention, particularly when the compositions include emulsions.

A wide variety of emulsifiers are useful herein and include those selected from the group consisting of anionic emulsifiers, nonionic emulsifiers, cationic emulsifiers, amphoteric emulsifiers, and combinations thereof.

Preferred emulsifiers are selected from the group consisting of anionic emulsifiers selected from the group consisting of ammonium lauroyl sarcosinate, sodium trideceth sulfate, sodium lauroyl sarcosinate, ammonium laureth sulfate, sodium laureth sulfate, ammonium lauryl sulfate, sodium lauryl sulfate, ammonium cocoyl isethionate, sodium cocoyl isethionate, sodium lauroyl isethionate, sodium cetyl sulfate, sodium monolauryl phosphates, ethoxylated monoalkyl phosphates, sodium cocoglyceryl ether sulfonate, sodium C₉-C₂₂ soap, and combinations thereof; nonionic emulsifiers selected from the group consisting of lauramine oxide, cocoamine oxide, decyl polyglucose, lauryl polyglucose, sucrose cocoate, C12-14 glucosamides, sucrose laurate, and combinations thereof; cationic emulsifiers selected from the group consisting of fatty amines, di-fatty quaternary amines, tri-fatty quaternary amines, imidazolinium quaternary amines, and combinations thereof; amphoteric emulsifiers selected from the group consisting of disodium lauroamphodiacetate, sodium lauroamphoacetate, cetyl dimethyl betaine, cocoamidopropyl betaine, cocoamidopropyl hydroxy sultaine, and combinations thereof.

### Crosslinked Silicone Polymers

The composition of the present invention may optionally include a polymer that is nonlinear in nature. Suitable polymers for inclusion in the claimed compositions include, but are not limited to polysiloxanes that are crosslinked organopolysiloxane polymer gel networks. For instance, particularly well-suited crosslinked organopolysiloxane polymer gel networks are formed from polymerization of an epoxy functional organosiloxane in the presence of an acid catalyst. The organopolysiloxane polymer is a crosslinked organopolysiloxane polymer gel network selected from non-emulsifying polymer gel networks, emulsifying polymer gel networks, and combinations thereof. Specific examples of such are described in U.S. Patent 6,531,540 B1, U.S. Patent 6,538,061 B2, U.S. Patent 6,444,745 B1, U.S. Patent 6,346,583 B1, US Patent 5,654,362, US Patent 5,811,487, US Patent 5,880,210, US Patent 5,889,108, US Patent 5,929,164, US Patent 5,948,855, US Patent 5,969,035, US Patent 5,977,280, US Patent 6,080,394, US Patent 6,168,782, US Patent 6,177,071, US Patent 6,200,581, US Patent 6,207,717, US Patent 6,221,927, US Patent 6,221,979, US Patent 6,238,657, and US Patent 4,987,169.

Suitable organopolysiloxane polymer network powders include vinyl dimethicone/methicone silesquioxane crosspolymers like Shin-Etsu's KSP-100, KSP-101, KSP-102, KSP-103, KSP-104, KSP-105, hybrid silicone powders that contain a fluoroalkyl group like Shin-Etsu's KSP-200, and hybrid silicone powders that contain a phenyl group such as Shin-Etsu's KSP-300; and Dow Coming's DC 9506.

Preferred organopolysiloxane compositions are dimethicone/vinyl dimethicone crosspolymers. Such dimethicone/vinyl dimethicone crosspolymers are supplied by a variety of suppliers including Dow Coming (DC 9040 and DC 9041), General Electric (Velvesil 125), General Electric (SFE-839), Shin Etsu (KSG-15, 16, 18 [dimethicone /phenyl vinyl dimethicone crosspolymer] and KSG-21 [dimethicone copolyol crosspolymer]), Grant Industries (Gransil^{™} line of materials), lauryl dimethicone/vinyl dimethicone crosspolymers supplied by Shin Etsu (e.g., KSG-41, KSG-42, KSG-43, and KSG-44), lauryl dimethicone/ dimethicone copolyol crosspolymers also supplied by Shin-Etsu (e.g., KSG-31, KSG-32, KSG-33, and KSG-34), and Wacker (Belsil RG-100) Additional polymers from Shin-Etsu which are suitable for use in the present invention include KSG-210, -310, 320, 330, and 340. Crosslinked organopolysiloxane polymer gel networks useful in the present invention and processes for making them are further described in US Patent 4,970,252 to Sakuta et al., issued November 13, 1990; US Patent 5,760,116 to Kilgour et al., issued June 2, 1998; US Patent 5,654,362 to Schulz, Jr. et al. issued August 5, 1997; and Japanese Patent Application JP 61-18708, assigned to Pola Kasei Kogyo KK.

### ASSOCIATED METHODS

Applicants have found that the cosmetic kit of the present invention is particularly useful for use as a multi-step facial foundation product. It is expected, however, that a skilled artisan is capable of envisioning the appropriate cosmetic benefit agent of those disclosed herein that are commensurate with the method of use being disclosed. The methods of use for the cosmetic kit that is disclosed and claimed herein include, but are not limited to: 1) methods of increasing the substantivity of a cosmetic active to skin; 2) methods of moisturizing skin; 3) methods of improving the natural appearance of skin; 4) methods of applying a color cosmetic to skin; 5) methods of providing antiperspirant efficacy to skin; 6) methods of preventing, retarding, and/or treating wrinkles; 7) methods of providing UV protection to skin; 8) methods of preventing, retarding, and/or controlling the appearance of oil; 9) methods of modifying the feel and texture of skin; 10) methods of providing even skin tone; 11) methods of preventing, retarding, and/or treating the appear of spider vessels and varicose veins; 12) methods of masking the appearance of vellus hair on skin; 13) methods of concealing blemishes and/or imperfections in human skin, including acne, age spots, freckles, moles, scars, under eye circles, birth marks, post-inflammatory hyperpigmentation, etc.; and 14) methods of preventing, retarding, and/or treating malodor of a mammal. Each of the methods discussed herein involve sequential topical application of a first and second composition to the skin. Each of the claimed methods may, however, involve additional steps that focus on the use of additional compositions that include other cosmetic benefit agents.

### EXAMPLES

The following examples further describe and demonstrate embodiments within the scope of the present invention. In the following examples, all ingredients are listed at an active level.

### EXAMPLES OF THE FIRST COMPOSITION

### Examples 1-5

Oil-in-water first compositions including astringents, shine control absorbents, anti-redness/yellowness pearl pigments, vitamins, and sunscreen actives.

| **Ingredient** | **Ex 1** | **Ex 2** | **Ex 3** | **Ex 4** | **Ex 5** |
|---|---|---|---|---|---|
| **Phase A:** | | | | | |
| Water | qs | qs | qs | qs | qs |
| Glycerin | 5.00 | 5.00 | 5.00 | 5.00 | 5.00 |
| Disodium EDTA | 0.10 | 0.10 | 0.10 | 0.10 | 0.10 |
| Methylparaben | 0.25 | 0.25 | 0.25 | 0.25 | 0.25 |
| **Phase B:** | | | | | |
| Octylmethoxycinnamate | - | - | - | 6.00 | - |
| Octocrylene | - | - | - | - | 1.50 |
| Avobenzone | - | - | - | - | 2.50 |
| Octyl Salicylate | - | - | - | - | 4.00 |
| Phenylbenzimidazole | | | | | |
| Sulfonic acid | - | - | - | - | 1.00 |
| Isohexadecane | 3.00 | 3.00 | 3.00 | 2.00 | - |
| Isopropyl Isostearate | 2.00 | 10.00 | 10.00 | 2.00 | 2.00 |
| Cetyl Alcohol | 1.00 | 1.00 | 1.00 | 1.00 | 1.00 |
| Stearic Acid | 0.20 | 0.20 | 0.20 | 0.20 | 0.20 |
| PEG-100 Stearate | 0.10 | 0.10 | 0.10 | 0.10 | 0.10 |
| Cetearyl Glucoside | 0.20 | 0.20 | 0.20 | 0.20 | 0.20 |
| Zinc Oxide | - | - | - | 4.50 | - |
| Ethylparaben | 0.15 | 0.15 | 0.15 | 0.15 | 0.15 |
| Propylparaben | 0.10 | 0.10 | 0.10 | 0.10 | 0.10 |
| **Phase C:** | | | | | |
| Titanium Dioxide | | | | | |
| Nylon-12 | 3.00 | 2.00 | 2.00 | 2.00 | 2.00 |
| Polyurethane spheres¹ | 2.00 | 2.00 | 2.00 | 2.00 | 2.00 |
| Absorbent Particles² | 1.00 | 4.00 | | 2.00 | 2.00 |
| Mica and Titianium | | | | | |
| Dioxide³ | 3.00 | - | - | - | - |
| Aluminum | | | | | |
| Chlorohydrate | - | - | 5.00 | - | - |
| **Phase D:** | | | | | |
| Polyacrylamide⁴ | 1.25 | 1.25 | 3.00 | 2.00 | 2.00 |
| **Phase E:** | | | | | |
| Sodium Hydroxide | 0.03 | 0.03 | 0.03 | 0.03 | 0.03 |
| **Phase F:** | | | | | |
| Niacinamide | - | - | - | 3.5 | 3.5 |
| D-Panthenol | - | - | - | 0.5 | 0.5 |
| Tocopherol Acetate | - | - | - | 0.5 | 0.5 |
| **Phase G:** | | | | | |
| Benzyl Alcohol | 0.25 | 0.25 | 0.25 | 0.50 | 0.50 |
| Fomes Officinalis | | | | | |
| extract⁵ | 5.00 | 3.00 | 3.00 | - | - |
| Dimethicone & | | | | | |
| Dimethiconol | 2.00 | 3.00 | 3.00 | 2.00 | 2.00 |
| Phase H: | | | | | |
| Acrylates/C12-22 | | | | | |
| Alkylmethacrylate | | | | | |
| Copolymer⁶ | - | 2.50 | 2.50 | - | - |

| | | | | | |
|---|---|---|---|---|---|
| ¹Polyurethane spheres available as BPD500T from Kobo Products ²Particles for absorbing sebum and sweat including, but not limited to, Silica in the form of Silica Shells or MSS-500/3H4 from Kobo Products, Allyl Methacrylate crosspolymer in the form of Polypore E200 or L200 from Amcol Beauty Solutions, Magnesium Aluminometasilicate in the form of Sebumase from Miyoshi Kasei, or absorbent starches such as Natrasorb Bath or Natrasorb HFB from National Starch ³Mica and Titanium Dioxide pearl pigments including, but not limited to, Timiron Silk Green or Timiron Super Green for Anti-redness or Timiron Silk Blue or Timiron Super Blue for anti-yellowness from Rona. ⁴Polyacrylamide with C13-C14 Isoparaffin and Laureth-7 available as Sepigel 305 from Seppic. ⁵Astringent Fomes officinalis extract available dispersed in 1,3 Butylene Glycol as LS8865 from Laboratoires Serobiologique. ⁶Acrylates/C12-22 Alkylmethacrylates copolymer available as Allianz OPT from International Specialty Products. | | | | | |

Combine the Phase A ingredients and heat to 75°C. Combine the Phase B ingredients and heat to 75°C. Both Phase A and B should be mixed with a low shear propeller or turbine to ensure phase homogeneity. Once both Phase A and B have reached 75°C, combine these two phases and emulsify with high shear mixer at 8000-10000rpm for 10 minutes. Add the Phase C ingredients to the emulsion and mix with a propeller or turbine mixer for 5-10 minutes, ensuring a good vortex is created to disperse the solids and break up large agglomerates. If necessary, the mixing of phase C into Phase A/B can be supplemented with a high shear mixer at 5000-10000rpm and an anchor-type mixer with sidewall scrapers. Once the solids have been dispersed, start cooling the emulsion while mixing with a propeller or turbine mixer and side scrapers. Once the emulsion temperature has reached 60°C, add the Phase D polyacrylamide thickener and mix with a propeller or turbine mixer for 5-10 minutes, ensuring a good vortex is created to disperse the polymer. Cool the emulsion to 55°C and add Phase E. Cool to 50°C and add phase F & G. Continue mixing with the propeller or turbine mixer while cooling the emulsion to 35-40°C. Once the emulsion reaches 35-40°C, the product can be transferred into an appropriate container for storage.

### Examples 6-8

### Water-in-silicone first compositions

| **Ingredient** | **Ex 6** | **Ex 7** | **Ex 8** |
|---|---|---|---|
| **Phase A:** | | | |
| Water | 20.00 | 20 | 20.00 |
| Glycerin | 10.00 | 10.00 | - |
| Disodium EDTA | 0.10 | 0.10 | 0.10 |
| Fomes Officinalis extract | 5.00 | 5.00 | 5.00 |
| Methylparaben | 0.25 | 0.25 | 0.25 |
| Ethanol | - | - | 10.00 |
| Benzyl Alcohol | 0.25 | 0.25 | 0.25 |
| **Phase B:** | | | |
| Cyclopentasiloxane | qs | qs | qs |
| Acrylates/Dimethicone copolymer¹ | 5.00 | 5.00 | 5.00 |
| Cyclomethicone & Dimethicone Crosspolymer² | 25.00 | 20.00 | 42.00 |
| Dimethicone copolyol crosspolymer³ | 0.50 | 0.50 | - |
| Cyclopentsiloxane & Dimethicone Copolyol⁴ | 9.50 | 9.50 | - |
| Ethylparaben | 0.15 | 0.15 | 0.15 |
| Propylparaben | 0.10 | 0.10 | 0.15 |
| Aluminum Chlorohydrate | - | 10.00 | - |
| Silicone Glycol copolymer | - | - | 2.50 |
| Polyoxyethylene Lauryl ether | - | | 0.50 |
| Polydimethylsiloxane⁵ | | | 10.00 |
| **Phase C:** | | | |
| Polymethylsilsesquioxane | 3.00 | 3.00 | 3.00 |
| Absorbent particles⁶ | 4.00 | 4.00 | 4.00 |

| | | | |
|---|---|---|---|
| ^{1,} Acrylates/Dimethicone copolymer such as KP545 from Shin Etsu. ²Cyclomethicone & Dimethicone Crosspolymer such as, but not limited to, DC9040 Silicone Elastomer blend from Dow Coming. ³Dimethicone copolyol cross polymer such as KSG-21, KSG-210 or KSP-104 self emulsifying elastomers from Shin Etsu. ⁴Polydimethylsiloxane, such as, but not limited to DC200 1cst or DC2-1184 from Dow Cornming ⁵cyclopentasiloxane & Dimethicone copolyol in the form of DC5225C formulation aid. ⁶Particles for absorbing sebum and sweat including, but not limited to, Silica in the form of Silica Shells, Silica Shells-SH or MSS-500/3H4 from Kobo Products, Allyl Methacrylate crosspolymer in the form of Polypore E200 or L200 from Amcol Beauty Solutions or Magnesium Aluminometasilicate in the form of Sebumase from Miyoshi Kasei. | | | |

Combine phase A and mix until homogenous with a propeller or turbine mixer. Combine the ingredients of Phase B and mix until homogenous with a high shear mixer at 5000-10000rpm. Combine Phase A with Phase B and emulsify with high shear mixer at 5000-10000rpm for 10-20 minutes. Do not allow the temperature of the emulsion to increase above 40°C. Add Phase C to the emulsion and mix with a propeller or turbine until homogenous. A high shear mixer may also be used if needed to break up particle agglomerates. Once particles are fully dispersed, allow the emulsion to cool to ambient temperature before transferring to an appropriate container.

### Example 9

### A pressed powder first composition.

| **Ingredient** | **Ex 9** |
|---|---|
| **Phase A:** | |
| Talc | qs |
| Mica | 18.00 |
| Sericite | 29.00 |
| Titanium Dioxide | 12.00 |
| Nylon-12 | 2.00 |
| Anhydrous Silicic Acid | 2.70 |
| Propylparaben | 0.10 |
| Methylparaben | 0.30 |
| Sodium Dehydroacetate | 0.10 |
| Absorbent Particles¹ | 5.00 |
| Aluminum Chlorohydrate | 5.00 |
| **Phase B:** | |
| Dimethicone and Trimetylsiloxysilicate | 6.5 |
| Dioctyl Succinate | 0.80 |
| Octyl Hydroxystearate | 1.00 |
| Tocopherol Acetate | 0.006 |
| Octyl Methoxycinnamate | 3.00 |

| | |
|---|---|
| ¹Particles for absorbing sebum and sweat including, but not limited to, Silica in the form of Silica Shells, Silica Shells-SH or MSS-500/3H4 from Kobo Products, Allyl Methacrylate crosspolymer in the form of Polypore E200 or L200 from Amcol Beauty Solutions, Magnesium Aluminometasilicate in the form of Sebumase from Miyoshi Kasei, or absorbent starches such as Natrasorb Bath or Natrasorb HFB from National Starch. | |

Combine Phase A ingredients with bulk mixing (e.g. Ribbon Blender, Double cone blender or by hand) followed by high shear mixing (e.g. hammer mill, pulveriser or chopping blades) to break down any particle agglomerates. Combine Phase B ingredients with a propeller mixer and heat to 75°C. Add Phase B ingredients to phase A and disperse with a combination of bulk and high shear mixing.

### Example 10

### A solid emulsion that is suitable for use as the first composition

| **Ingredient** | **Ex 10** |
|---|---|
| **Phase A:** | |
| Cyclomethicone | qs |
| Tridecyl Isonoanoate | 2.00 |
| Tocopherol Acetate | 18.00 |
| Cyclopentasiloxane and Dimethicone copolyol | 29.00 |
| Sorbitan Isostearate | 2.00 |
| **Phase B:** | |
| Water | 20.00 |
| Fomes Officinalis Extract | 5.00 |
| 1, 3 Butylene Glycol | 2.00 |
| Disodium EDTA | 0.10 |
| Methylparaben | 0.20 |
| **Phase C:** | |
| Absorbent Particles | 5.00 |
| Mica and Titanium Dioxide | 3.00 |
| Aluminum Chlorohydrate | 5.00 |
| **Phase D:** | |
| Ozokerite Wax | 2.00 |
| Propylparaben | 0.25 |
| Candelilla Wax | 2.00 |

| | |
|---|---|
| ¹Particles for absorbing sebum and sweat including, but not limited to, Silica in the form of Silica Shells, Silica Shells-SH or MSS-500/3H4 from Kobo Products, Allyl Methacrylate crosspolymer in the form of Polypore E200 or L200 from Amcol Beauty Solutions, Magnesium Aluminometasilicate in the form of Sebumase from Miyoshi Kasei, or absorbent starches such as Natrasorb Bath or Natrasorb HFB from National Starch. | |

Combine Phase A ingredients and mix at 20-30°C with a low shear propeller or turbine mixer until homogenous. Combine Phase B ingredients and mix at 20-30°C with a propeller or turbine mixer until homogenous. Combine Phase A and Phase B with a high shear mixer at 5000-10000rpm. Add Phase D ingredients to the emulsion and mix with low shear until dispersed. Add Phase D ingredients to the emulsion and heat to 85°C. At 85°C, mix at 5000-10000rpm for 10 minutes to ensure all waxes and solids are dispersed. Cool the emulsion to 65°C ready for pouring into the appropriate storage containers.

### EXAMPLES OF THE SECOND COMPOSITION

### Examples 11-14

### Water-in-silicone second compositions

| INGREDIENT | EXAMPLE 11 | EXAMPLE 12 | EXAMPLE 13 | EXAMPLE 14 |
|---|---|---|---|---|
| PHASE A | | | | |
| CYCLOPENTASILOXANE & DIMETHICONE COPOLYOL | 8.000 | 8.000 | 8.000 | 5.600 |
| SILICONE-MODIFIED ACRYL RESIN¹ | 14.820 | 7.700 | 14.809 | 5.390 |
| POLYACRYLATES-g-POLYSILOXANE RESIN² | - | 20.391 | - | 14.274 |
| TRIDECYL NEOPENTANOATE³ | 6.000 | 6.000 | 6.000 | 4.200 |
| CETYL DIMETHICONE COPOLYOL⁴ | 2.000 | 2.000 | 2.000 | 1.400 |
| CYCLOHEXASILOXANE | 2.000 | - | 2.000 | - |
| CYCLOPENTASILOXANE | 3.275 | 11.031 | 5.786 | 7.797 |
| PHASE B | | | | |
| ETHOXYLATED C10-16 ALCOHOLS⁵ | 0.500 | 0.500 | 0.500 | 0.500 |
| PROPYLPARABEN | 0.150 | 0.150 | 0.150 | 0.150 |
| PHASE C | | | | |
| YELLOW PIGMENT | 0.737 | 0.737 | 0.737 | 0.595 |
| RED PIGMENT | 0.238 | 0.238 | 0.238 | 0.195 |
| BLACK PIGMENT | 0.130 | 0.130 | 0.130 | 0.106 |
| MICRONIZED TITANIUM DIOXIDE | 0.250 | 0.250 | 0.250 | 0.175 |
| BORON NITRIDE TREATED STARCH⁶ | 3.500 | 3.500 | 3.500 | 2.450 |
| TITANIUM DIOXIDE | 8.250 | 8.250 | 8.250 | 5.775 |
| POLYMETHYLSILSESQUIOXA NE⁷ | 1.500 | 1.500 | 1.500 | 1.050 |
| HEXAMETHYLENE DIISOCYANATE/POLYPORPYL ENE/POLYCAPROLACTONE CROSSPOLYMER (AND) SILICA⁸ | 1.500 | 1.500 | 1.500 | 1.050 |
| DlMETHICONE/VINYLDIMETH ICONE CROSSPOLYMER⁹ | 2.000 | 1.500 | 3.000 | 1.050 |
| MIXED CYCLOMETHICONES AND EPOXY GEL¹⁰ | - | - | - | 30.000 |
| PHASE D | | | | |
| DEIONIZED WATER | 40.000 | 22.973 | 35.000 | 15.583 |
| SILICA SHELLS¹¹ | 2.500 | 1.000 | 4.000 | 0.700 |
| PHENOXYETHANOL | 0.250 | 0.250 | 0.250 | 0.250 |
| TRISODIUMEDTA | 0.100 | 0.100 | 0.100 | 0.100 |
| SODIUM CHLORIDE | 2.000 | 2.000 | 2.000 | 1.400 |
| SODIUM DEHYDROACETATE MONOHYDRATE | 0.300 | 0.300 | 0.300 | 0.210 |
| TOTALS | 100.000 | 100.000 | 100.000 | 100.000 |

| | | | | |
|---|---|---|---|---|
| 1. Shin-Etsu - KP-545 2. 3M - SA-70 3. ISP - Ceraphyl 55 4. DeGussa Goldsmith - Abil WE-09 5. Rhodia - Rhodasurf L-7/ 90 6. National Starch - Dry Flo Elite BN 7. GE - Tospearl 145A 8. Kobo - BPD-500T 9. Dow Coming - DC 9506 Powder 10. GE - Velvesil -1111-19-372 11. Kobo - Silica Shells | | | | |

Procedure: Combine Phase DeIonized Water and Silica Shells of Phase D Ingredients and mix with propeller or dispersator until homogeneity is achieved. Add remaining Phase D ingredients and continue propeller or dispersator blending. Combine Phase A ingredients in jacketed vessel and begin mixing with rotor stator mill. Recirculate cold water through jacketed vessel. Blend Phase B ingredients together for ten minutes. Add blended Phase B into Phase A ingredients. Add Phase C Ingredients into Phase AB. Shear Phase ABC until it is completely deagglomerated and pigments have been reduced to their primary particle size. Emulsify Phase D into Phase ABC under moderate shear. Blend Phase ABCD with sweep wall mixing until uniform. Store in suitably sized vessel.

### Example 15

A powder foundation that is suitable for use as the second composition.

| **Phase A** | **WT%** |
|---|---|
| Talc | 23.90 |
| Mica | 17.66 |
| Mica (sericite) | 29.04 |
| Titanium Dioxide | 11.60 |
| Nylon-12 | 1.76 |
| Silica | 2.64 |
| Propylparaben, NF | 0.10 |
| Methylparaben, NF | 0.30 |
| Sodium Dehydroacetate, NF | 0.10 |
| Red Iron Oxide | 0.43 |
| Black Iron Oxide | 0.29 |
| Yellow Iron Oxide | 0.50 |
| **Phase B** | |
| Dimethicone (and) Trimethylsiloxysilicate | 6.43 |
| Dioctyl Succinate | 0.80 |
| Octyl Hydroxystearate | 0.70 |
| Cholesterol Hydroxystearate | 1.05 |
| Tocopherol | 0.01 |
| Octyl Methoxycinnamate | 2.69 |
| | 100 |

Combine Phase A ingredients with bulk mixing (e.g. Ribbon Blender, Double cone blender or by hand) followed by high shear mixing (e.g. hammer mill, pulveriser or chopping blades) to break down any particle agglomerates. Combine Phase B ingredients with a propeller mixer and heat to 75°C. Add Phase B ingredients to phase A and disperse with a combination of bulk and high shear mixing.

### Examples 16-17

Water in silicone second composition examples:

| | Ingredient | Example 16 | Example 17 |
|---|---|---|---|
| A1 | Cyclopentacyloxane and dimethicone copolyol | 9.000 | 9.000 |
| A2 | Tridecyl Neopentanoate | 6.300 | 6.300 |
| A3 | Decamethylcyclopentacyloxane | 14.543 | 14.543 |
| A4 | Polyethylene Glycol (7) Lauryl Ether | 0.500 | 0.500 |
| A5 | Propylparaben | 0.150 | 0.150 |
| B1 | Titanium Dioxide (And) Polyglyceryl-4 Isostearate (And) Cetyl Dimethicone Copolyol (And) Hexyl Laurate (And) Isopropyl Titanium Triisostearate¹ | 12.062 | 12.062 |
| B2 | Iron Oxide (CI 77492) (And) Polyglyceryl-4 Isostearate (And) Cetyl Dimethicone Copolyol (And) Hexyl Laurate (And) Isopropyl Titanium Triisostearate¹ | 1.382 | 1.382 |
| B3 | Iron Oxide (CI 77491) (And) Polyglyceryl-4 Isostearate (And) Cetyl Dimethicone Copolyol (And) Hexyl Laurate (And) Isopropyl Titanium Triisostearate¹ | 0.314 | 0.314 |
| B4 | Iron Oxide (CI 77499) (And) Polyglyceryl-4 Isostearate (And) Cetyl Dimethicone Copolyol (And) Hexyl Laurate (And) Isopropyl Titanium Triisostearate¹ | 0.189 | 0.189 |
| B5 | Aluminum Starch Octenylsuccinate⁴ | 5.000 | 5.000 |
| C1 | Deionized water | 17.510 | 17.510 |
| C2 | Polyvinylpyrrolidone | 1.500 | 1.500 |
| C3 | Phenoxyethanol | 0.250 | 0.250 |
| C4 | Trisodium edetate Edetate | 0.100 | 0.100 |
| C5 | Sodium Chloride | 1.000 | 1.000 |
| C6 | Sodium dehydroacetate monohydrate | 0.200 | 0.200 |
| D1 | Cyclopentasiloxane (and) C30-45 Alkyl Cetearyl Dimethicone Crosspolymer² | ---- | 12.000 |
| D2 | Cyclopentasiloxane (and) C30-45 Alkyl Cetearyl Dimethicone Crosspolymer (and) titanium dioxide (and) iron oxides³ | 30.000 | 18.000 |

| | | | |
|---|---|---|---|
| 1. Kobo Products - ITT Coated Pigments 2. GeneraL Electric Silicones-Velvesil 125 3. General Electric Silicones- 1111-21-937 4. National Starch - Dry Flo Elite BN | | | |

1. Combine Phase C in plastic bucket. Provide maximum prop mixer blending without air incorporation.
2. Add Phase A ingredients 1- 6 to stainless steel jacketed vessel and begin high shear mixing.
3. Add Phase B ingredients to Phase A and begin milling on HIGH for approximately 30 minutes.
4. Add phase C to phase AB in vessel with homogenization. Continue homogenizing until batch uniformity is visually achieved.
5. Add Phase D ingredients and homogenize until uniformity is achieved

### Examples 18-21

Water in silicone first composition examples:

| | Ingredient | Example 18 | Example 19 | Example 20 | Example 21 |
|---|---|---|---|---|---|
| A1 | Dodecamethyl cyclohexasiloxane | 12.450 | 12.000 | 11.550 | 12.000 |
| A2 | Silica ¹ | 2.000 | 2.000 | 2.000 | 2.000 |
| A3 | Tridecylneopentanoate | 5.000 | 6.000 | 5.000 | 6.000 |
| A4 | Isopropyl isostearate | ---- | ---- | 3.000 | --- |
| A6 | Dimethicone (500 cSt) | ---- | ---- | --- | 30.00 |
| A7 | Cyclopentasiloxane (and) dimethicone copolyol | 8.000 | 8.900 | --- | 8.900 |
| A8 | Cyclopentasiloxane (and) C30-45 Alkyl Cetearyl Dimethicone Crosspolymer (87.5%)² | 22.000 | 30.000 | 24.000 | |
| A9 | Cyclopentasiloxane (and) C30-45 Alkyl Cetearyl Dimethicone Crosspolymer (70.0%)² | 9.500 | ---- | ---- | ---- |
| A10 | Cyclopentasiloxane (and) C30-45 Alkyl Cetearyl Dimethicone Crosspolymer (and) PEG/PPG 20/15 Dimethicone ³ | --- | ---- | 12.000 | ---- |
| B1 | Zinc Oxide | 3.000 | 3.00 | 3.000 | 3.00 |
| C1 | Propylparaben | 0.200 | 0.200 | 0.200 | 0.200 |
| C2 | Octyl methoxycinnimate | 6.000 | 6.000 | 6.000 | 6.000 |
| D1 | SD alcohol | 8.500 | 8.500 | 10.000 | 8.500 |
| D2 | Deionized water | 16.050 | 16.500 | 15.950 | 16.500 |
| D3 | Sodium chloride | 1.000 | 1.000 | -- | 1.000 |
| D4 | Methylparaben | 0.200 | 0.200 | 0.200 | 0.200 |
| D5 | Glycerin | 4.000 | 4.000 | 5.000 | 4.000 |
| D6 | Trisodium edetate | 0.100 | 0.100 | 0.100 | 0.100 |
| D7 | Phemoxyehanol | 0.400 | --- | 0.400 | ---- |
| E1 | Acrylates/C12-22 Alkylmethacrylate ⁴ | 1.600 | 1.600 | 1.600 | 1.600 |

| | | | | | |
|---|---|---|---|---|---|
| 1. Kobo Products - Silica Shells 2. General Electric Silicones - Velvesil 125 3. General Electric Silicones - 1120-21-381 4. Rohm and Haas - Allianz OPT | | | | | |

1. Combine ingredients D1 and D5 into large stainless steel breaker. Provide maximum prop mixer blending without air incorporation.
2. Add Phase A and B ingredients to a stainless steel vessel. Mix mix at high shear for 15 minutes or until uniformity is achieved.
3. Add Phase C ingredients into a separate beaker. Manually mix until paraben is dissolved.
4. Add Phase C to batch. Continue high shear until homogenious.
5. Once Phase ABC are uniform insert homogenizer then add Phase D and continue homogenization until uniformity is achieve.
6. Add Phase E and blend with prop mixer until uniform in appearance and consistency

### Examples 22-23

Silicone in water second composition examples:

| | Ingredient | Example 22 | Example 23 |
|---|---|---|---|
| A1 | Decamethylcyclopentasiloxane | 9.145 | 9.145 |
| A2 | Dodecamethyl cyclohexasiloxane | 2.065 | 2.065 |
| A3 | Tridecyl Neopentanoate | 8.000 | 8.000 |
| A4 | PCA Dimethicone | 2.000 | 2.000 |
| A5 | Propylparaben | 0.150 | 0.150 |
| A6 | Arachadyl Behenate | 0.300 | 0.300 |
| A7 | Stearyl Alcohol | 0.750 | 0.750 |
| B1 | Titanium Dioxide (And) Polyglyceryl-4 Isostearate (And) Cetyl Dimethicone Copolyol (And) Hexyl Laurate (And) Isopropyl Titanium Triisostearate ¹ | 9.075 | 9.075 |
| B2 | Iron Oxide (CI 77492) (And) Polyglyceryl-4 Isostearate (And) Cetyl Dimethicone Copolyol (And) Hexyl Laurate (And) Isopropyl Titanium Triisostearate ¹ | 0.810 | 0.810 |
| B3 | Iron Oxide (CI 77491) (And) Polyglyceryl-4 Isostearate (And) Cetyl Dimethicone Copolyol (And) Hexyl Laurate (And) Isopropyl Titanium Triisostearate ¹ | 0.262 | 0.262 |
| B4 | Iron Oxide (CI 77499) (And) Polyglyceryl-4 Isostearate (And) Cetyl Dimethicone Copolyol (And) Hexyl Laurate (And) Isopropyl Titanium Triisostearate ¹ | 0.143 | 0.143 |
| B5 | Silica ² | --- | 2.00 |
| C1 | Deionized Water | 54.000 | 52.000 |
| C2 | Methylparaben | 0.200 | 0.200 |
| C3 | Phenoxyethanol | 0.500 | 0.500 |
| C4 | Hydroxypropyl Starch Phosphate | 2.000 | 2.000 |
| C5 | Glycerin | 2.250 | 2.250 |
| C6 | Butylene Glycol | 2.250 | 2.250 |
| C7 | Polyvinylpyrrolidone | 1.000 | 1.000 |
| C8 | Trisodium Edetate | 0.100 | 0.100 |
| C9 | Sucrose Palmitate (and) Glyceryl Stearate (and) Glyceryl Stearate Citrate (and) Sucrose (and) Mannan (and) Xanthan Gum³ | 2.000 | 2.000 |
| C10 | Aluminum Starch Octenylsuccinate ⁴ | 3.000 | 3.00 |

1. Kobo Products - ITT Coated Pigments
2. Kobo Products - Silica Shells
3. Uniquima - Arlatone V-175
4. National Starch - Dry Flo Elite BN

1. Combine ingredients C1 and C9 with maximum propeller.
2. Add Phase C ingredients 2,3,5,6,7,8 and 10. Provide maximum prop mixer blending without air incorporation
3. Heat Phase C to 70 - 80C. Once batch reaches 70-80C add 50% C4.
4. Add Phase A ingredients 1- 5 to separate vessel and begin homogenizing batch.
5. Heat Phase A to 70 - 80C.
5. Add Phase B to Phase A shear on HIGH for approximately 20-30 minutes.
6. Once Phase AB reaches 70-80C add Phase A ingredients 6 -7.
7. Transfer Phase AB to Phase C while prop mixing. Blend until uniform in appearance.
8. Homogenize batch with high shear. Add remaining 50% C4. Maintain until uniformity is achieved.

## Claims

1. A cosmetic kit suitable for application as a multi-step facial foundation product, said kit
**characterized by**:
a. a first composition that comprises :
i. a safe and effective amount of a first cosmetic benefit agent; and
ii. a first cosmetically acceptable carrier; and
b. a second composition that comprises:
i. a safe and effective amount of one or more colorants having a refractive index greater than 2.0 ; and
ii. a second cosmetically acceptable carrier;
wherein said second composition is topically applied to facial skin after said first composition,
wherein the first composition is an oil-in-water emulsion, **characterized in that** the second composition is a water-in-silicone emulsion.

2. The cosmetic kit of claim 1 wherein said first cosmetic benefit agent is selected from the group consisting of absorbents, anti-acne actives, antiperspirant actives, anti-wrinkle actives, artificial tanning agents, astringents, film forming agents, hydrophilic conditioning agents, hydrophobic conditioning agents, light diffusers, desquamating agents, skin lightening agents, skin soothing/heating agents, skin thickeners, sunscreen actives, vitamin compounds, yellowness- reducing particles, redness-reducing particles, and combinations thereof.

3. The cosmetic kit of claim 1 wherein said colorant is selected from the group consisting of zinc oxide, titanium dioxide, iron oxides, guanine, carmine, aluminum powder, ultramarines, bismuth oxychloride, chromium oxide green, chromium hydroxide green, ferric ferrocyanide, manganese violet, titanium dioxide, titanated mica, iron oxide titanated mica, zinc oxide, mica, ferric ammonium ferrocyanide, dihydroxyacetone, guaiazulene, pyrophyllite, bronze powder, copper powder, aluminum stearate, calcium stearate, lactofavin, magnesium stearate, zinc stearate, capsanthin/capsorubin, bentonite, barium sulfate, calcium carbonate, calcium sulfate, carbon black, magnesium carbonate, magnesium silicate, colored silica, chalk, and combinations thereof.

4. The cosmetic kit of claim 1 wherein said first composition, said second composition, or both said first composition and said second composition further comprise a safe and effective amount of one or more film forming agents, preferably said film forming agents are selected from the group consisting of hydrophilic film forming agents, water-dispersible hydrophobic film forming agents, and combinations thereof, preferably said film forming agents are hydrophobic.

5. The cosmetic kit of claim 1 wherein said first composition is free of pigments.

6. The cosmetic kit of claim 1 wherein said second composition further comprises cosmetic benefit agents selected from the group consisting of absorbents, anti-acne actives, antiperspirant actives, anti-wrinkle actives, artificial tanning agents, astringents, film forming agents, hydrophilic conditioning agents, hydrophobic conditioning agents, light diffusers, desquamating agents, skin lightening agents, skin soothing/healing agents, skin thickeners, sunscreen actives, vitamin compounds, yellowness-reducing particles, redness-reducing particles, and combinations thereof.

7. The cosmetic kit of claim 1 wherein said first and second compositions are in product forms selected from the group consisting of liquids, solid emulsions, creams, lotions, powders, and combinations thereof.

8. The cosmetic kit of claim 1 wherein said kit further comprises one or more additional compositions that are sequentially applied after said first and second compositions.

9. The cosmetic kit of claim 1 wherein said first composition, said second composition, or both said first composition and said second composition further comprise a crosslinked silicone polymer and one or more film forming agents.

## Patentansprüche

1. Kosmetikset, das zur Anwendung als Mehrschritt-Gesichtsgrundierungsprodukt geeignet ist, wobei das Set **gekennzeichnet ist durch**:
a. eine erste Zusammensetzung, die Folgendes umfasst:
i. eine sichere und wirksame Menge eines ersten vorteilhaften kosmetischen Wirkstoffes; und
ii. einen ersten kosmetisch annehmbaren Träger; und
b. eine zweite Zusammensetzung, die Folgendes umfasst:
i. eine sichere und wirksame Menge eines oder mehrerer Farbmittel mit einem Brechungsindex von mehr als 2,0; und
ii. einen zweiten kosmetisch annehmbaren Träger;
wobei die zweite Zusammensetzung nach der ersten Zusammensetzung topisch auf die Gesichtshaut aufgetragen wird, wobei die erste Zusammensetzung eine Öl-in-Wasser-Emulsion ist, **dadurch gekennzeichnet, dass** die zweite Zusammensetzung eine Wasser-in-Silikon-Emulsion ist.

2. Kosmetikset nach Anspruch 1, wobei der erste vorteilhafte kosmetische Wirkstoff ausgewählt ist aus der Gruppe, bestehend aus Absorptionsmitteln, aknehemmenden Wirkstoffen, schweißhemmenden Wirkstoffen, Antifaltenwirkstoffen, künstlichen Bräunungsmitteln, Adstringenzien, Filmbildnern, hydrophilen Konditionierungsmitteln, hydrophoben Konditionierungsmitteln, Lichtstreuern, Abschilferungsmitteln, Hautaufhellern, hautberuhigenden/-heilenden Wirkstoffen, Hautverdickungsmitteln, Sonnenschutzmitteln, Vitaminverbindungen, Gelbstichigkeit reduzierenden Teilchen, Rotstichigkeit reduzierenden Teilchen und Kombinationen davon.

3. Kosmetikset nach Anspruch 1, wobei das Farbmittel ausgewählt ist aus der Gruppe, bestehend aus Zinkoxid, Titandioxid, Eisenoxid, Guanin, Karmin, Aluminiumpulver, Ultramarinen, Bismutoxychlorid, Chromoxidgrün, Chromhydroxidgrün, Eisen(III)-ferrocyanid, Manganviolett, Titandioxid, titaniertem Glimmer, titaniertem Eisenoxidglimmer, Zinkoxid, Glimmer, Eisen(III)-ammonium-ferrocyanid, Dihydroxyaceton, Guaiazulen, Pyrophyllit, Bronzepulver, Kupferpulver, Aluminiumstearat, Calciumstearat, Lactofavin, Magnesiumstearat, Zinkstearat, Capsanthin/Capsorubin, Bentonit, Bariumsulfat, Calciumcarbonat, Calciumsulfat, Kohleschwarz, Magnesiumcarbonat, Magnesiumsilicat, farbigem Silica, Kreide und Kombinationen davon.

4. Kosmetikset nach Anspruch 1, wobei die erste Zusammensetzung, die zweite Zusammensetzung oder sowohl die erste Zusammensetzung als auch die zweite Zusammensetzung ferner eine sichere und wirksame Menge eines oder mehrerer Filmbildner umfassen, wobei die Filmbildner vorzugsweise ausgewählt sind aus der Gruppe, bestehend aus hydrophilen Filmbildner, wasserdispergierbaren hydrophoben Filmbildner und Kombinationen davon, wobei die Filmbildner vorzugsweise hydrophob sind.

5. Kosmetikset nach Anspruch 1, wobei die erste Zusammensetzung frei von Pigmenten ist.

6. Kosmetikset nach Anspruch 1, wobei die zweite Zusammensetzung ferner vorteilhafte kosmetische Wirkstoffe umfasst, die ausgewählt sind aus der Gruppe, bestehend aus Absorptionsmitteln, aknehemmenden Wirkstoffen, schweißhemmenden Wirkstoffen, Antifaltenwirkstoffen, künstlichen Bräunungsmitteln, Adstringenzien, Filmbildner, hydrophilen Konditionierungsmitteln, hydrophoben Konditionierungsmitteln, Lichtstreuern, Abschilferungsmitteln, Hautaufhellern, hautberuhigenden/-heilenden Wirkstoffen, Hautverdickungsmitteln, Sonnenschutzmitteln, Vitaminverbindungen, Gelbstichigkeit reduzierenden Teilchen, Rotstichigkeit reduzierenden Teilchen und Kombinationen davon.

7. Kosmetikset nach Anspruch 1, wobei die erste und die zweite Zusammensetzung in Produktformen vorliegen, die ausgewählt sind aus der Gruppe, bestehend aus Flüssigkeiten, Feststoffemulsionen, Cremes, Lotionen, Pulvern und Kombinationen davon.

8. Kosmetikset nach Anspruch 1, wobei das Set ferner eine oder mehrere zusätzliche Zusammensetzungen umfasst, die sequenziell nach der ersten und der zweiten Zusammensetzung aufgetragen werden.

9. Kosmetikset nach Anspruch 1, wobei die erste Zusammensetzung, die zweite Zusammensetzung oder sowohl die erste Zusammensetzung als auch die zweite Zusammensetzung ferner ein vernetztes Silikonpolymer und einen oder mehrere Filmbildner umfassen.

## Revendications

1. Trousse cosmétique appropriée pour une application comme produit de type fond de teint facial multi-étapes, ladite trousse étant **caractérisée par** :
a. une première composition qui comprend :
i. une quantité sûre et efficace d'un premier agent bénéfique cosmétique ; et
ii. un premier véhicule cosmétiquement acceptable ; et
b. une deuxième composition qui comprend :
i. une quantité sûre et efficace d'un ou de plusieurs colorants présentant un indice de réfraction supérieur à 2,0 ; et
ii. un deuxième véhicule cosmétiquement acceptable ;
dans laquelle ladite deuxième composition est appliquée topiquement sur la peau faciale après ladite première composition, où la première composition est une émulsion huile dans l'eau, **caractérisée en ce que** la deuxième composition est une émulsion eau dans silicone.

2. Trousse cosmétique selon la revendication 1, dans laquelle ledit premier agent bénéfique cosmétique est choisi dans le groupe constitué d'absorbants, principes actifs anti-acnéiques, principes actifs antiperspirants, principes actifs anti-rides, agents de bronzage artificiels, agents astringents, agents filmogènes, agents de conditionnement hydrophiles, agents de conditionnement hydrophobes, agents de diffusion de la lumière, agents de desquamation, agents de décoloration de la peau, émollients/agents de cicatrisation, agents d'épaississement dermique, principes actifs d'écran solaire, composés vitaminiques, particules réduisant le jaunissement, particules réduisant la rougeur, et leurs combinaisons.

3. Trousse cosmétique selon la revendication 1, dans laquelle ledit colorant est choisi dans le groupe constitué d'oxyde de zinc, dioxyde de titane, oxydes de fer, guanine, carmin, poudre d'aluminium, outremers, oxychlorure de bismuth, vert d'oxyde de chrome, vert d'hydroxyde de chrome, ferrocyanure ferrique, violet de manganèse, dioxyde de titane, mica titanaté, mica titanaté à l'oxyde de fer, oxyde de zinc, mica, ferrocyanure ferrique d'ammonium, dihydroxyacétone, guaïazulène, pyrophyllite, poudre de bronze, poudre de cuivre, stéarate d'aluminium, stéarate de calcium, lactofavine, stéarate de magnésium, stéarate de zinc, capsanthine/capsorubine, bentonite, sulfate de baryum, carbonate de calcium, sulfate de calcium, noir de carbone, carbonate de magnésium, silicate de magnésium, silice colorée, craie, et leurs combinaisons.

4. Trousse cosmétique selon la revendication 1, dans laquelle ladite première composition, ladite deuxième composition, ou à la fois ladite première composition et ladite deuxième composition comprennent, en outre, une quantité sûre et efficace d'un ou de plusieurs agents filmogènes, lesdits agents filmogènes étant de préférence choisis dans le groupe constitué d'agents filmogènes hydrophiles, agents filmogènes hydrophobes hydrodispersibles et de leurs combinaisons, de préférence, lesdits agents filmogènes sont hydrophobes.

5. Trousse cosmétique selon la revendication 1, dans laquelle ladite première composition est exempte de pigments.

6. Trousse cosmétique selon la revendication 1, dans laquelle ladite deuxième composition comprend, en outre, des agents bénéfiques cosmétiques choisis dans le groupe constitué d'absorbants, principes actifs anti-acnéiques, principes actifs antiperspirants, principes actifs anti-rides, agents de bronzage artificiels, astringents, agents filmogènes, agents de conditionnement hydrophiles, agents de conditionnement hydrophobes, agents de diffusion de la lumière, agents de desquamation, agents de décoloration de la peau, émollients/agents de cicatrisation, agents d'épaississement dermique, principes actifs d'écran solaire, composés vitaminiques, particules réduisant le jaunissement, particules réduisant la rougeur, et de leurs combinaisons.

7. Trousse cosmétique selon la revendication 1, dans laquelle ladite première et ladite deuxième composition sont sous des formes de produits choisies dans le groupe constitué de liquides, émulsions solides, crèmes, lotions, poudres et de leurs combinaisons.

8. Trousse cosmétique selon la revendication 1, dans laquelle ladite trousse comprend, en outre, une ou plusieurs compositions supplémentaires qui sont appliquées séquentiellement après ladite première et ladite deuxième compositions.

9. Trousse cosmétique selon la revendication 1, dans laquelle ladite première composition, ladite deuxième composition, ou à la fois ladite première composition et ladite deuxième composition comprennent, en outre, un polymère de silicone réticulé et un ou plusieurs agents filmogènes.
